# EUROPEAN PATENT SPECIFICATION

(11) **EP 4 447 841 B1**
(45) Date of publication and mention of the grant of the patent: **15.10.2025**
(21) Application number: 22830871.4
(22) Date of filing: 08.12.2022
(51) Int. Cl.: A61B 34/20, A61B 34/30, A61B 90/00, G16H 50/70, G16H 40/63, G16H 20/40

(54) **SYSTEM AND METHOD FOR ESTIMATING AND VISUALIZING TRAJECTORIES OF ROBOTICALLY CONTROLLED INTERVENTIONAL DEVICE**
SYSTEM UND VERFAHREN ZUM SCHÄTZEN UND VISUALISIEREN VON BEWEGUNGSBAHNEN EINER ROBOTISCH GESTEUERTEN EINGRIFFSVORRICHTUNG
SYSTÈME ET PROCÉDÉ D'ESTIMATION ET DE VISUALISATION DE TRAJECTOIRES D'UN DISPOSITIF D'INTERVENTION À COMMANDE ROBOTIQUE

(30) Priority: 16.12.2021 US 202163290117 P; 23.02.2022 EP 22158160
(43) Date of publication of application: 23.10.2024
(73) Proprietor: Koninklijke Philips N.V., 5656 AG Eindhoven (NL)
(72) Inventor: FOTOUHI, Javad, 5656 AG Eindhoven (NL); BALICKI, Marcin Arkadiusz, 5656 AG Eindhoven (NL); SINHA, Ayushi, 5656 AG Eindhoven (NL); HAASE, Christian, 5656 AG Eindhoven (NL)
(74) Representative: Philips Intellectual Property & Standards
(86) International application number: PCT/EP2022/084886
(87) International publication number: WO 2023/110598

(56) References cited:
- WO-A1-2020/173814
- US-A1- 2018 325 602
- US-A1- 2020 315 572

## Description

### BACKGROUND

Robotic systems used for interventional procedures (e.g., endovascular procedures) are operated by a user operating a robotic controller that includes interface devices, such as a joystick controller. The robotic systems control movement of various interventional devices, such as catheters or guidewires. The motion and behavior of the interventional devices in a subject (patient) depend on the input that the user triggers from the robotic controller, as well as the shape, properties and position of the interventional devices relative to the subject's anatomy.

In robot-assisted interventional procedures, steering bendable interventional devices through high curvatures, bifurcations and tortuous anatomical structures (e.g., vessels) may be difficult and complicated, which may result in multiple failed attempts that lead to damage to the anatomical structure (e.g., hemorrhage), increased procedure time, increased exposure of the subject to imaging radiation, and/or the need to change the interventional device. Conventional solutions have no way to visualize or otherwise predict potential trajectories of the interventional device when given different inputs to the robotic system. Consequently, the user may apply suboptimal input, resulting in multiple failed attempts.

Therefore, what is needed is a robotic system that predicts a trajectory of an interventional device during an interventional procedure based on control inputs by the user, and provides a display that shows dynamic lines indicating where the control inputs will position the interventional device before actually moving the interventional device.

WO 2020/173814 A1 describes a training data collection method for an interventional device including a portion of an interventional device and sensors adapted to provide position and/or orientation and/or shape information with at least a part of the sensors being affixed to said portion of device. The method involves controlling one or more motion variables of the interventional device in accordance with a predefined data point pattern, and determining, from shape data derived from said information, an estimating of a pose of said device portion and an estimating of a positioning motion of the interventional device. The method further involves a storage of a temporal data sequence for the interventional device derived from the estimated pose of the end-effector for each data point, the estimated positioning motion of the interventional device for each data point, and the motion variable(s) of the interventional device or each data point.

US 2018/325602 A1 describes a transperinealprostate intervention device comprising a prostate intervention instrument, a transrectal ultrasound probe, and a mechanical or optical coordinate measurement machine.

US 2020/315572 A1 describes a framework for image-based probe positioning receiving a current image from a probe. The current image is acquired by the probe within a structure of interest.

### SUMMARY

The present invention is defined by the independent claims. Further embodiments are provided in the dependent claims.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 shows an illustrative interventional device with a predicted trajectory and associated uncertainty margins on a display, according to a representative embodiment.
FIG. 2 is a simplified block diagram of a system for estimating and visualizing trajectories of a robotically controlled interventional device on a display including a graphical user interface (GUI), according to a representative embodiment.
FIG. 3 is a simplified block diagram showing an example implementation of training an encoder-decoder neural network architecture, according to a representative embodiment.
FIG. 4 is a flow diagram showing a method of estimating and visualizing trajectories of a robotically controlled interventional device on a display including a GUI, according to a representative embodiment.
FIG. 5 is a flow diagram of a method of determining and visualizing trajectories of a robotically controlled interventional device on a display including a GUI, according to another representative embodiment.
FIG. 6 is a flow diagram of a method of controlling a robotically controlled interventional device by adjusting visualized trajectories of the interventional device on a display including a GUI, according to another representative embodiment.
FIG. 7 is a simplified block diagram showing an example implementation of training an encoder-decoder neural network architecture, according to a representative embodiment.
FIG. 8 is a simplified block diagram showing another example implementation of training an encoder-decoder neural network architecture, according to a representative embodiment.
FIG. 9 is a simplified block diagram of an encoder-decoder neural network model configured to predict device trajectory with dimension preserving architecture, according to a representative embodiment.
FIG. 10 is a simplified block diagram of an encoder-decoder neural network model configured to predict device trajectory with dimension varying architecture, according to a representative embodiment.
FIG. 11 is a flow diagram of a method of estimating and visualizing trajectories of a robotically controlled interventional device using discontinuous imaging, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. Moreover, descriptions of well-known apparatuses and methods may be omitted so as to not obscure the description of the example embodiments. Such methods and apparatuses are within the scope of the present disclosure.

Generally, the various embodiments described herein provide a system and method that enable a user (interventionalist) of a robotic system to visualize future (predicted) trajectories of a robotically controlled interventional device based on preliminary (intended) control inputs to the robotic system before the control input is applied or executed. The user is therefore able to adjust the preliminary control inputs based on the predicted trajectories before triggering motion of the interventional device. Predicting the trajectory based on the preliminary inputs reduces the number of failed attempts, lowers the risk of puncture or other damage to tissue, lowers exposure to radiation, and generally improves the usability of the robotic system. A robot controller estimates and visualizes the predicted trajectory of the robotically controlled interventional device before the preliminary input is triggered in an uncalibrated robot-patient setting. The estimates and visualizations are based on planned inputs by the user (without actuating the robot), and the predicted trajectory of the interventional device is overlaid on a recent fluoroscopic image.

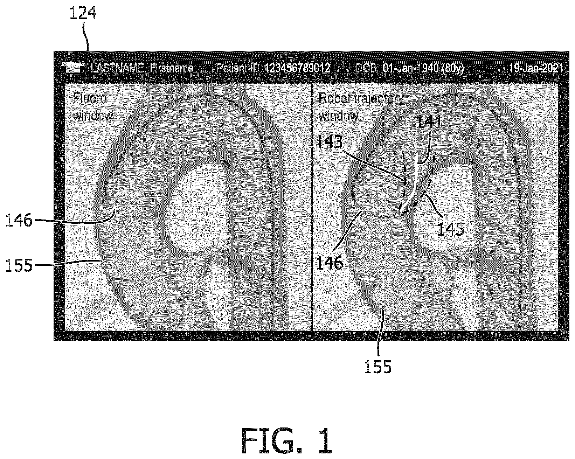

FIG. 1 shows an illustrative interventional device with a predicted trajectory and associated uncertainty margins on a display, according to a representative embodiment.

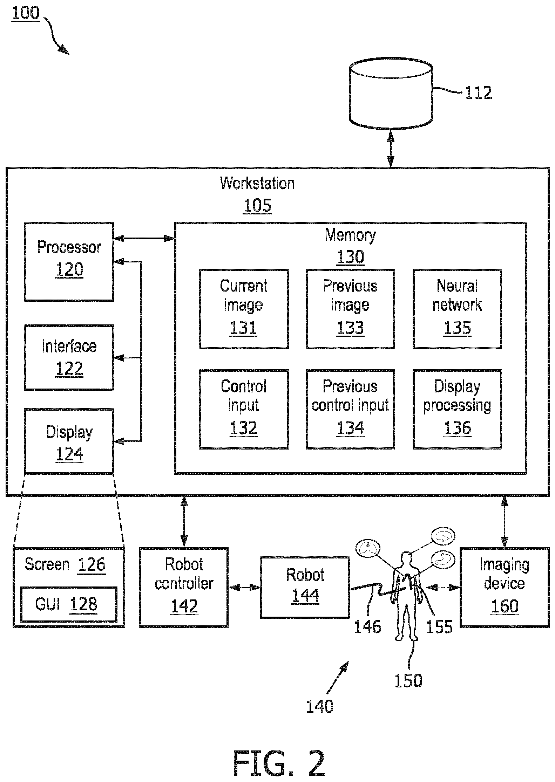

Referring to FIG. 1, a display 124 shows fluoroscopic images of an interventional device 146 configured for insertion into an anatomical structure 155 of a subject, such as a vessel or an artery, for example. The interventional device 146 is guided by a robot controller and a robot, such as robot controller 142 and robot 144 discussed below with reference to FIG. 2. The interventional device 146 may be any compatible medical instrument capable of robotic control, such as a catheter, a guidewire, an endoscope, a camera, a surgical tool, or the like. The image on the left shows the current position of the interventional device 146 within the anatomical structure 155. The image on the right shows a predicted trajectory of the interventional device 146 within the anatomical structure 155 based on control inputs for controlling the robot to guide the interventional device 146 if the control inputs were to be sustained. Control inputs include user inputs provided to the robot through a user interface and the robot controller for steering and otherwise maneuvering the robot in the anatomical structure 155, and state information provided by the robot controller for indicating the state of the robotic system responsive to the user input. For example, the control inputs may be untriggered, where the user enters the desired user inputs but does not yet trigger the robot controller to implement the entered control inputs. The user inputs may include directional and velocity control signals for the robot to navigate the interventional device 146, and the state information may include physical positioning of the robot, as well as kinematics with regard articulation, rotation, translation, velocity and acceleration of the robot, for example.

In the depicted embodiment, the predicted trajectory is indicated by a centerline 141, and the estimated uncertainty associated with the predicted trajectory is indicated by uncertainty margins indicated by outer-lines 143 and 145 on either side of the centerline 141. The centerline 141 extends longitudinally from a distal end of the interventional device 146 in its current position along an estimated path that the interventional device 146 would follow if the control inputs are sustained (i.e., triggered) by the user. The outer-lines 143 and 145 increasingly depart from the centerline 141 the further the centerline 141 extends away from the current position of the interventional device 146. The outer-lines 143 and 145 thus indicate increasing uncertainty in the prediction the more the predicted trajectory extends from the current position. Alternatively, or in addition, the uncertainty margins may be indicated by color coded pixels defining margins along the centerline of the predicted trajectory, e.g., within the bounds of the outer-lines 143 and 145, where the outer-lines 143 and 145 may or may not be displayed together with the color coded pixels.

The display 124 may be continuously updated as additional images are acquired, and/or as the user changes or fine tunes the user inputs. The predicted trajectory and associated uncertainty may be determined using a neural network module, which has been trained using previously acquired images of the interventional device 146 together with corresponding previous control inputs used to position the interventional device 146 as shown in the previous images, as discussed below.

FIG. 2 is a simplified block diagram of a system for estimating and visualizing trajectories of a robotically controlled interventional device on a display including a GUI, according to a representative embodiment.

Referring to FIG. 2, system 100 includes a workstation 105 for implementing and/or managing the processes described herein with regard to controlling movement of an interventional device 146 within an anatomical structure 155 of a subject (patient) 150. The workstation 105 includes one or more processors indicated by processor 120, one or more memories indicated by memory 130, a user interface 122 and a display 124. The processor 120 interfaces with a robotic system 140 through a controller interface (not shown), where the robotic system 140 includes a robot controller 142 and a robot 144. The robot controller 142 is configured to control movement of the robot 144 in response to user inputs received through the user interface 122 or a separate robotic control interface (not shown). The robot 144 is attached to or integrated with the interventional device 146. The robot 144 may include segments, joints, servo motors and other control features operable for moving and positioning the interventional device 146 in multiple degrees of freedom (DOFs) in response to control signals received from the robot controller 142. In the depicted embodiment, the robot controller 142 is shown separately from processor 120 in the workstation 105 for purposes of illustration. It is understood, though, that the all or part of the functionality of the robot controller 142 may be incorporated into the processor 120, or vice versa, without departing from the scope of the present teachings.

The processor 120 also interfaces with an imaging device 160 through an imaging interface (not shown). The imaging device 160 may be any of various types of medical imaging device/modality, including a fixed or mobile C-arm fluoroscopy system, an X-ray imaging device, a computerized tomography (CT) scan device, a magnetic resonance (MR) imaging device, a positron emission tomography (PET) scan device, or an ultrasound imaging device, for example. The imaging device 160 may include single or multiple imaging modalities.

The memory 130 stores instructions executable by the processor 120. When executed, the instructions cause the processor 120 to implement one or more processes for estimating and visualizing trajectories of robotically controlled interventional device 146 on the display 124. For purposes of illustration, the memory 130 is shown to include software modules, each of which includes the instructions corresponding to an associated capability of the system 100.

The processor 120 is representative of one or more processing devices, and may be implemented by field programmable gate arrays (FPGAs), application specific integrated circuits (ASICs), a digital signal processor (DSP), a general purpose computer, a central processing unit, a graphical processing unit, a computer processor, a microprocessor, a microcontroller, a state machine, programmable logic device, or combinations thereof, using any combination of hardware, software, firmware, hard-wired logic circuits, or combinations thereof. Any processing unit or processor herein may include multiple processors, parallel processors, or both. Multiple processors may be included in, or coupled to, a single device or multiple devices. The term "processor" as used herein encompasses an electronic component able to execute a program or machine executable instruction. A processor may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 130 may include main memory and/or static memory, where such memories may communicate with each other and the processor 120 via one or more buses. The memory 130 may be implemented by any number, type and combination of random access memory (RAM) and read-only memory (ROM), for example, and may store various types of information, such as software algorithms, artificial intelligence (AI) machine learning models, and computer programs, all of which are executable by the processor 120. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted.

The system 100 may also include a database 112 for storing information that may be used by the various software modules of the memory 130. For example, the database 112 may include image data from previously obtained images of the subject 150 and/or of other similarly situated subjects having the same or similar interventional procedures as the subject 150. The stored image data may be used for training an AI machine learning model, such as a neural network model, for example, as discussed below. The database 112 may be implemented by any number, type and combination of RAM and ROM, for example. The various types of ROM and RAM may include any number, type and combination of computer readable storage media, such as a disk drive, flash memory, EPROM, EEPROM, registers, a hard disk, a removable disk, tape, CD-ROM, DVD, floppy disk, Blu-ray disk, USB drive, or any other form of storage medium known in the art. The database 112 comprises tangible storage mediums for storing data and executable software instructions and is non-transitory during the time data and software instructions are stored therein. The database 112 may be secure and/or encrypted, or unsecure and/or unencrypted. For purposes of illustration, the database 112 is shown as a separate storage medium, although it is understood that it may be combined with and/or included in the memory 130, without departing from the scope of the present teachings.

The processor 120 may include or have access to an artificial intelligence (AI) engine, which may be implemented as software that provides artificial intelligence (e.g., neutral network models) and applies machine learning described herein. The AI engine may reside in any of various components in addition to or other than the processor 120, such as the memory 130, an external server, and/or the cloud, for example. When the AI engine is implemented in a cloud, such as at a data center, for example, the AI engine may be connected to the processor 120 via the internet using one or more wired and/or wireless connection(s).

The user interface 122 is configured to provide information and data output by the processor 120, the memory 130 and/or the robot controller 142 to the user and/or for receiving information and data input by the user. That is, the user interface 122 enables the user to enter data and to control or manipulate aspects of the processes described herein, and also enables the processor 120 to indicate the effects of the user's control or manipulation. All or a portion of the user interface 122 may be implemented by a graphical user interface (GUI), such as GUI 128 viewable on the display 124, discussed below. The user interface 122 may include one or more interface devices, such as a mouse, a keyboard, a trackball, a joystick, a microphone, a video camera, a touchpad, a touchscreen, voice or gesture recognition captured by a microphone or video camera, for example.

The display 124 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 124 includes a screen 126 for viewing internal images of the subject 150, along with various features described herein to assist the user in accurately and efficiently reading the images, as well as the GUI 128 to enable the user to interact with the displayed images and features. The user is able to personalize the various features of the GUI 128, discussed below, by creating specific alerts and reminders, for example.

Referring to the memory 130, the various modules store sets of data and instructions executable by the processor 120 to estimate and visualize predicted trajectories of the robotically controlled interventional device 146. Current image module 131 is configured to receive and process a current (first) image of the anatomical structure 155 of the subject 150 for display on the display 124. The current image is the image currently being viewed by the user during the interventional procedure. The current image may be received in real time from the imaging device 160 during a contemporaneous current imaging session of the subject 150. Alternatively, the current image may be an image of the subject 150 previously acquired in the current imaging session, where the imaging has been paused, for example, to reduce exposure of the subject 150 to radiation. Likewise, the current image may be retrieved from the database 112, which stores images obtained during previous imaging session(s) or earlier in the current imaging session (from single or multiple imaging modalities). The current image is displayed on the screen 126 to enable analysis by the user and navigation of the robot 144.

Control input module 132 is configured to receive user inputs from the user via the user interface 122 for controlling the robot 144 to guide movement of the interventional device 146 and state information via the robot controller 142 as control inputs. According to various embodiments, the control inputs are initially untriggered, in that the user enters the user inputs without them being executed by the robot controller 142. In this manner, the processor 120 is able to predict the effect of the control inputs on the trajectory of the interventional device 146, as discussed below, prior to the robot controller 142 controlling the robot 144 to actually move the interventional device 146 in response to the control inputs. This enables the user to determine whether the untriggered control inputs are appropriate, e.g., in that their execution would move the interventional device 146 to a desired position by a desired route and would otherwise avoid damaging the anatomical structure 155, before action is taken to implement the control inputs. The control inputs include maneuvering instructions, such as articulation, rotation, translation, distance, velocity, and acceleration of the robot 144, for example, for moving the interventional device 146 in the anatomical structure 155.

Previous image module 133 is configured to receive previous images of the subject 150, for example, from the imaging device 160 and/or the database 112. The previous images include images of the subject 150 acquired earlier in the current imaging session and/or images of the subject 150 acquired during previous imaging sessions involving the same type of interventional procedure. The images may be acquired from a single or multiple imaging modalities. In an embodiment, the previous image module 133 may also receive previous images of other similarly situated subjects having the same or similar interventional procedures as the subject 150 having the same type of interventional procedure. The previous images are used to train a neural network model, discussed below.

Previous control input module 134 is configured to receive previous control inputs associated with the previous images of the previous image module 133. That is, for each previous image, the previous control input module 134 receives the corresponding control inputs provided to the robot controller 142 used to maneuver the interventional device to the position captured by that previous image. The associations between the previous control inputs and the previous images are maintained.

Prediction model module 135 is configured to implement prediction model, such as a neural network model, for example, for predicting trajectories of the interventional device 146 based on control inputs for controlling movements of the robot 144 via the robot controller 142. The prediction model may also estimate the uncertainties associated with the predicted trajectories. For example, the prediction model may determine uncertainty margins (e.g., outer-lines 143 and 145) that are displayed along with a centerline (e.g., centerline 141) on the display 124 to indicate the uncertainty as a function of distance from a current known position of the interventional device 146, as discussed above. In an embodiment, the uncertainty margins may be extended to a volumetric model, for example, a three-dimensional convolutional neural network (CNN). In various embodiments, all or part of the processes provided by the prediction model module 135 may be implemented by an AI engine, for example, mentioned above.

The prediction model module 135 provides training of the prediction model with regard to predicting trajectories of the interventional device 146 and associated uncertainties based on training data. The training data includes previous images of interventional device(s) and corresponding control inputs for guiding the interventional device(s) as shown in the previous images. For example, the training data may include previous images of the interventional device 146 in the anatomical structure 155 of the subject 150 during the current imaging session and corresponding control inputs to the robot 144 used to guide the interventional device 146 to positions shown in the previous images. The previous images may be retrieved from the previous image module 133 and the corresponding control inputs may be retrieved from the previous control input module 134. Alternatively, the training data may include previous images and corresponding control inputs from previous imaging sessions involving the subject 150, or involving other similarly situated subjects, as discussed above. In an embodiment, current images retrieved from the current image module 131 and corresponding control inputs retrieved from the control input module 132 may also be included in the training data, which enables training of the prediction model on-the-fly. Accordingly, it is understood that discussion herein regarding training using previous images and corresponding control inputs likewise applies to training using current images and corresponding control inputs, without departing from the scope of the present teachings.

Training the prediction model may include capturing spatial context and temporal context of the previous images. The spatial context may be captured using convolutional layers, which consist of a sliding window or kernel representing a matrix of weights that slide over the input images and perform element-wise multiplication with the overlapping part of the input image and summing the results into an output feature map. The temporal context may be captured using temporal connections across layers, such as by using recurrent neural networks (RNNs), long-short term memory (LSTM), transformers, and the like. The prediction model training may be performed without supervision (unsupervised training) or with supervision (supervised training), where image and control input acquisitions may serve as outputs (labels) for previous image and control input acquisitions. Therefore, during the training phase, the prediction model will learn appropriate representations from retrospective data where previous image frames 1, ..., n-1 are used to predict trajectories of the interventional device(s) in the following m time points (image frames n, ..., n+m).

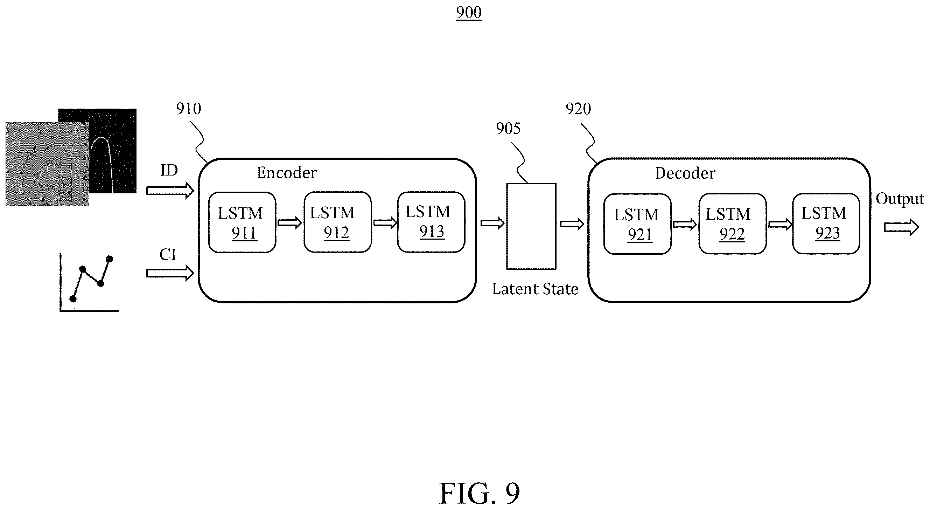

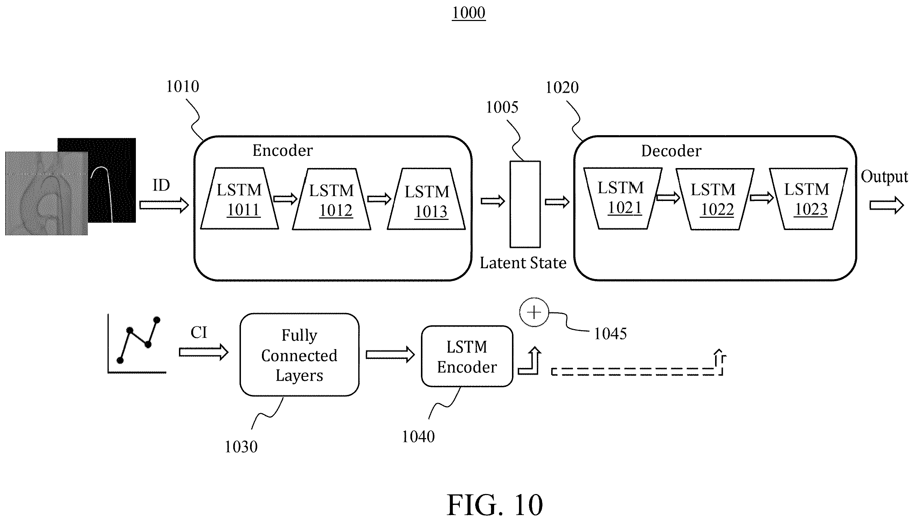

The previous image and previous control input data may be fed together in the earliest layer of a neural network model, an example of which is discussed below with reference to FIG. 9. The previous (and current) image data may include fluoroscopic images, or segmentation maps from different devices or anatomies within the image, for example. The previous (and current) control input data may include measurements from the user interface 122 and/or controller console of the robot controller 142, kinematics of the system with regard articulation, rotation and translation, as well as velocity and acceleration, device type and device stack used by the robot, number of devices actuated, and robot calibration parameters, for example. Alternatively, the previous image data may be fed in the earliest layer of a neural network model, and the previous control input data may be used at an intermediate or latent layer of the neural network model, acting as a transformation applied to the representation learned from the previous image frames 1, ..., n-1, an example of which is discussed below with reference to FIG. 10. In this case, the control input data may also be passed through a series of fully connected layers before merging with the convolutional network. The predicted output in the future image frames n, ..., n+m will produce different trajectories for different robot transformations or control input data. Another implementation may use two separate neural networks, one for the imaging data and one for robot control data. In this case, the two neural networks will share weights or feature maps at some intermediary layers. The training of the neural network model is performed iteratively, where at each iteration, a batch of corresponding previous image and corresponding previous control input data are fed into the neural network model. The training is preceded by minimizing a similarity loss, such as a binary-cross-entropy or intensity loss, as would be apparent to one skilled in the art.

In an embodiment, the prediction model is trained with regard to predicting trajectories of the interventional device based on training data from images of the interventional device and control inputs to the robot for guiding movement of the interventional device corresponding to the images. Training the prediction model may include receiving the training data; inputting at least one image of the interventional device in the anatomical structure into the prediction model; inputting at least one control input to the robot for guiding a next movement of the interventional device; generating a predicted trajectory of the interventional device using the prediction model; and adjusting parameters of the prediction model based on a difference between the predicted trajectory and a corresponding ground truth trajectory from the at least one image of the interventional device corresponding to the inputted robot control inputs. These steps are repeated until a stopping criterion is met. For example, the stopping criterion may be when the difference between the estimated trajectory of the interventional device and the ground truth trajectory of the interventional device is less than a predetermined threshold. Another stopping criterion may be distance between the tip of the trajectory (distal portion) to a target anatomical landmark, such as the trajectory tip near the ostium or near the aneurysm, for example.

In addition to training the prediction model, the prediction model module 135 predicts the trajectory of the interventional device 146 by applying a current image from the current image module 131 and control inputs from the control input module 132 to the trained prediction model. The output of the prediction model provides the predicted trajectory and the uncertainty of the predicted trajectory as a function of the distance the predicted trajectory extends from the current position of the interventional device 146. The distance or time in the future for which the predicted trajectory is generated may be a function of velocity of the interventional device 146 (per degree of freedom or linked to current projection). The gain may be adjusted by the user.

That is, the prediction model module 135 receives image data from the current image of the anatomical structure 155, showing a current position of the interventional device 146 with respect to the anatomical structure 155, and receives control inputs for controlling the robot 144 to guide future movement of the interventional device 146 from the current position. The control inputs are untriggered, so that the predicted trajectory may be determined before the robot controller 142 actually implements the control inputs. The control inputs may include current robot parameters and configuration space, such as joystick inputs, force, torque, and forward and inverse kinematics, for example. The prediction model module 135 may also input relevant segmentation maps associated with the current image. The prediction model provided by the prediction model module 135 predicts the trajectory of the interventional device 146 in the current image, as well as the associated uncertainty, by applying the image data and the untriggered control inputs to the trained prediction model, discussed above.

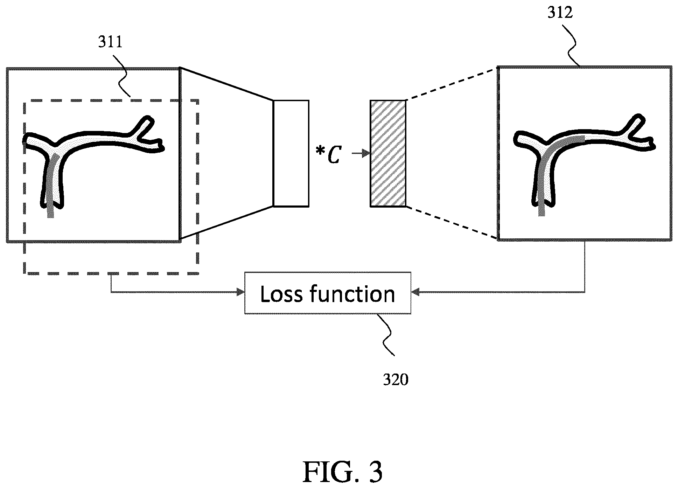

FIG. 3 is a simplified block diagram showing an example implementation of training an encoder-decoder neural network architecture, according to a representative embodiment. The encoder-decoder neural network architecture may be used where the current image is input into the encoder portion of the neural network, which generates a reduced dimension latent representation of the input image. The encoder and decoder portions may contain multiple blocks to capture temporal dependencies, such as RNNs, LSTMs, transformers, and the like. The untriggered control inputs may be applied to the latent representation to produce a transformed latent representation which can be decoded using the decoder portion of the neural network to generate the predicted image containing the predicted trajectory of the interventional device 146.

Referring to FIG. 3, training image 311 may be used as a ground truth image, to which known control inputs C have been applied. During training, the predicted image 312 may be compared to the ground truth image 311, for instance, where the predicted image 312 results from the application of the control inputs C on the current position of the interventional device 146. The comparison may be performed using a loss function 320, as discussed above, and the losses may be used to perform gradient descent to update neural network parameters over multiple iterations in order to minimize the computed loss function over the multiple iterations. By way of example, transforming auto-encoders, which is a similar concept, is described by G. E. Hinton et al. "Transforming auto-encoders" (ICANN 2011). The predicted trajectory and the uncertainty may also be fed back as input to the prediction model module 135 as training data to further train the neural network and assist with the predictions for subsequent untriggered control input data. When the predicted trajectory is determined to be acceptable and the untriggered control inputs are triggered, the resulting trajectory may also be fed back into the neural network as the new current image. In addition, the current image data and corresponding current untriggered control input(s) may be fed back as input to the neural network module 135 as training data for on-the-fly training, as discussed above.

The uncertainty may be estimated using a Monte Carlo dropout algorithm that approximates Bayesian inference for a deep Gaussian process, for example. To trigger dropout, a subset of the neurons is switched off during the forward pass. To compute uncertainty from the dropout, every incoming batch of data is passed through the neural network model multiple times (e.g., ten times). Every time, the dropout algorithm will provide a slightly different form of the neural network model that can subsequently result in another trajectory prediction by the neural network model. The outcomes are aggregated to compute upper and lower bounds for the predicted trajectory. These upper and lower bounds may be visualized on the current image, e.g., as outer-lines 143 and 145, as discussed below, and indicate the uncertainty for the predicted trajectory.

In an embodiment, roadmap images are incorporated into the prediction model. The roadmap images may include registered anatomical structural (e.g., vasculature) information from contrast angiograms and/or pre-procedural imaging. Boundaries of the anatomical structure 155 indicated by the roadmap images affect the expected shape of the interventional device 146. Accordingly, by incorporating the roadmap images, the prediction model may return a different predicted trajectory and uncertainty output, resulting in a different graphical representation of the predicted trajectory.

Display processing module 136 is configured to receive the predicted trajectory and the associated uncertainty, and to display the predicted trajectory and the associated uncertainty on the display 124 overlaid on the current image of the anatomical structure 155. For example, the display processing module 136 may be configured to cause the predicted trajectory to be displayed as a centerline (e.g., centerline 141) and the associated uncertainty to be displayed as uncertainty margins (e.g., outer-lines 143 and 145), as discussed above with reference to FIG. 1. **In** the event there are multiple coaxial interventional devices 146, each interventional device 146 has its own predicted trajectory display of the corresponding predicted trajectory, where these predicted trajectory displays are inherently coupled to the estimated behavior of both interventional devices 146.

When there is in and out of plane motion of the interventional device 146, the color scheme of predicted trajectory may be correlated with changes in magnitude and direction of the shape of the interventional device 146 along the predicted trajectory. For example, when the interventional device 146 is predicted to move toward a detector of the imaging device 160, the shape of the interventional device 146 is changing little in the current image while the robot 144 is translating the interventional device 146. **In** this case, the color of predicted trajectory may vary between a first color (e.g., more blue) when the interventional device 146 is expected to shape toward the detector, and a second color (e.g., more green) when the interventional device 146 is expected to shape away from the detector.

The display enables the user to determine whether the predicted trajectory is acceptable with regard to navigating the interventional device 146, taking into account the uncertainty margins. For example, the predicted trajectory may be acceptable when the predicted trajectory advances the interventional device 146 toward a target while avoiding contact with inner walls of the anatomical structure 155, and/or when the predicted trajectory substantially matches a desired trajectory of the interventional device 146. When a volumetric model is used, the display processing module 136 may display a projection of the uncertainty margins or the three-dimensional predicted trajectory overlaid on the current image.

When the predicted trajectory is acceptable, the user triggers the robotic system 140 to move the interventional device 146 in accordance with the control inputs. That is, the robot controller 142 may receive a trigger command, e.g., from the user interface 122, for activating the untriggered control inputs, and in response, control the robot 144 according to the triggered control inputs to guide movement of the interventional device 146. Ideally, the robot 144 moves the interventional device 146 along a path that matches the predicted trajectory, although the path may deviate from the predicted trajectory, particularly when there is relatively high uncertainty. In an embodiment, image data from additional images of the anatomical structure 155 may be received from the imaging device 160 after triggering the untriggered input, in which case an actual trajectory of the interventional device 146 may be displayed on the display 124 overlaid on the current image, along with the predicted trajectory, after triggering the untriggered input. Also, the interventional device 146 may be displayed overlaid on the current image while moving the interventional device 146 in accordance with the triggered input. Or, image data from additional (second) images of the anatomical structure 155 may be received from the imaging device 160 while controlling current movement of the interventional device 146 in accordance with the triggered control inputs, in which case the actual trajectory of the interventional device 146 may be overlaid on the additional images while controlling the current movement of the interventional device 146.

Alternatively, or in addition, the acceptability of the predicted trajectory may be determined automatically, e.g., by the processor 120. For example, the shape of the predicted trajectory may be captured using known image analysis techniques to provide a segmented predicted trajectory, and comparing segmented predicted trajectory to a desired trajectory for navigating the interventional device 146. For example, the predicted trajectory may be compared to a desired centerline or other datum in the anatomical structure 155. When the comparison is within a predetermined margin of error, the processor 120 verifies the predicted trajectory. In another example, the user may draw the desired trajectory on the GUI 128, and the segmented predicted trajectory may be compared to the desired trajectory to determine the acceptability of the predicted trajectory. The control inputs may then be triggered automatically, or by the user upon notification of the verified trajectory. In another example, the segmented predicted trajectory may be compared to a predetermined set of rules for navigating the interventional device 146. For example, the predicted trajectory may be acceptable when it advances the interventional device toward a target while avoiding contact with inner walls of the anatomical structure. In another example, the estimated trajectory may be compared to the desired trajectory by comparing the curves (e.g., based on thresholds or geodesic distances), by parameterizing the curves using polynomials and splines and comparing distances in the parametric space, or by comparing the distance between the tip of the estimated trajectory to a desired anatomical landmark.

When the predicted trajectory is not acceptable, the user may adjust the user inputs of the untriggered control inputs to the robot controller 142 for controlling the robot 144, which would guide the future movement of the interventional device 146 from its current position along an alternative predicted trajectory. In this case, the control input module 132 receives new user inputs from the user via the user interface 122 and state information from the robotic system 140 as the new control inputs. Again, the new control inputs are initially untriggered, in that the user enters the user inputs without them being executed by the robot controller 142 so that the processor 120 is able to predict the effect of the new control inputs on the trajectory of the interventional device 146 without the robotic system 140 actually moving the interventional device 146.

The prediction model module 135 receives the new control inputs, along with a current image of the interventional device 146 in the anatomical structure 155 provided by the current image module 131. The current image may be the same image used to predict the trajectory based on the previous control inputs, or the current image may be a more recently acquired image. The prediction model module 135 applies the new control inputs and the current image to the prediction model, which outputs a new (adjusted) predicted trajectory of the interventional device 146 and associated uncertainty. Again, the new predicted trajectory and the uncertainty may be fed back as input to the prediction model module 135 to make predictions regarding the future state.

When the new predicted trajectory is acceptable, the user triggers the robotic system 140 to move the interventional device 146 in accordance with the new control inputs. When the new predicted trajectory is not acceptable, process of providing new untriggered control inputs, outputting new predicted trajectories in response, and displaying the new predicted trajectories overlaid on the current image is repeated until an acceptable predicted trajectory is obtained, at which point the corresponding control inputs are triggered to move the interventional device 146 accordingly. This process is essentially continuous in that the new predicted trajectories are displayed substantially immediately following the receipt of the new untriggered control inputs. So, for example, the user may watch the effects that changes to the control inputs have on the displayed predicted trajectories at substantially the same time the changes to the control inputs are being made.

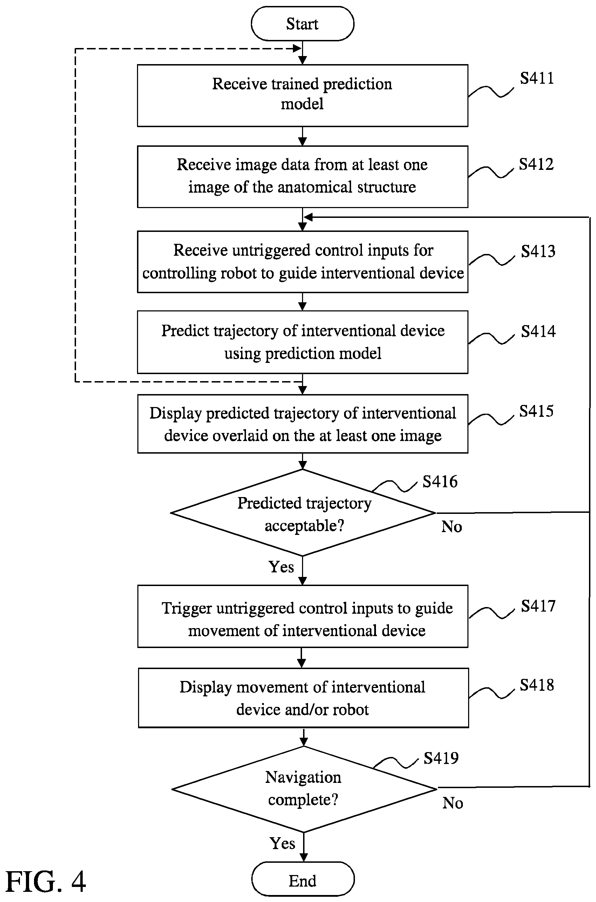

FIG. 4 is a flow diagram of a method of estimating and visualizing trajectories of a robotically controlled interventional device on a display including a GUI, according to a representative embodiment. The method may be implemented by the system 100, discussed above, under control of the processor 120 executing instructions stored as the various software modules in the memory 130, for example.

Referring to FIG. 4, the method includes receiving a prediction model in block S411, where the prediction model may be a neural network model, for example, and has been previously trained with regard to predicting trajectories of the interventional device and associated uncertainties. The training of the prediction model may be based on training data from previous images of the interventional device and corresponding control inputs to the robot for guiding movement of the interventional device as shown in the previous images. The training may be further based on training data from current images of the interventional device and corresponding control inputs to the robot for guiding movement of the interventional device as shown in the current images.

In block S412, image data is received from at least one image of the anatomical structure, where the at least one image is displayed on a display. The at least one image may be the current image, discussed above. The at least one image shows a current position of the interventional device with respect to the anatomical structure.

In block S413, at least one untriggered control input is received for controlling the robot to guide future movement of the interventional device from the current position. The at least one untriggered control input includes a user input that may be received via a user interface and state information that may be received from the robotic system. The user input may result from movement of one or more interface devices, such as a mouse, a keyboard, a trackball, a joystick, a microphone, a video camera, a touchpad, a touchscreen, for example. Since the at least one control inputs is untriggered, it has no immediate effect on movement of the robot or the interventional device controlled by the robot.

In block S414, a trajectory of the interventional device in the at least one image is predicted by applying the received image data and the at least one untriggered control input to the trained prediction model. That is, the trajectory of the interventional device may be predicted in the image by providing the received image data of the anatomical structure (first input) and the at least one untriggered control input corresponding to the received image data (second input) to the trained prediction model. The prediction model is configured with imaging of the interventional device and robot control parameters to predict trajectories of the interventional device based on the first and second inputs for guiding movement of the interventional device as shown in the image data of the first input. Along with the predicted trajectory, the trained prediction model also estimates uncertainty associated with the predicted trajectory. Generally, the uncertainty increases the further the predicted trajectory extends from the current position of the interventional device. Notably, in an embodiment, the predicted trajectory and the associated uncertainty may be returned to block S411, along with the corresponding untriggered control inputs, to further train the prediction model on-the-fly.

In block S415, the predicted trajectory of the interventional device is displayed on the display overlaid on the at least one image of the anatomical structure. In an embodiment, the estimated uncertainty associated with the predicted trajectory may also be displayed over laid on the at least one image. For example, the predicted trajectory may be shown as a centerline on the at least one image, and the associated uncertainty may be shown as outer-lines on either side of the centerline, as discussed above.

In block S416, it is determined whether the predicted trajectory has been identified as being an acceptable trajectory for purposes of continuing to navigate the interventional device in the anatomical structure. The determination may be made in response to input entered by the user via the user interface accepting or rejecting the predicted trajectory. Alternatively, or in addition, the acceptability of the predicted trajectory may be determined automatically. For example, the predicted trajectory may be determined automatically using known image analysis techniques to provide a segmented predicted trajectory, and comparing segmented predicted trajectory to a desired trajectory or to a predetermined set of rules for navigating the interventional device, as discussed above.

When the predicted trajectory is not acceptable (S416: No), the process returns to block S413, where new untriggered control inputs are received for controlling the robot. The new untriggered control inputs would guide future movement of the interventional device from the current position differently than the previous untriggered control inputs. Blocks S414 through S416 are then repeated in an attempt to identify an acceptable predicted trajectory.

When the predicted trajectory is acceptable (S416: Yes), the untriggered control inputs are triggered in block S417 to control the robot to guide movement of the interventional device according to the triggered control inputs. In an embodiment, block S417 may be effectively combined with block S416 where the predicted trajectory is determined to be acceptable in response to receiving a command triggering the untriggered control inputs.

Movement of the interventional device and/or the robot in response to the triggered control inputs may be displayed in block S418. The movement of the interventional device may be monitored using the display, enabling continued navigation of the interventional device through the anatomical structure. Actual movement of the interventional device may be displayed by acquiring additional images of the interventional device in real time during the interventional procedure. Alternatively, the movement of the interventional device may be simulated using the same image as a reference image, and applying the triggered control inputs to a navigation model for navigating the interventional device, where the navigation model is essentially the same as the prediction model that was used for the prediction (e.g., neural network model). In an embodiment, since the navigation model is essentially the same as the prediction model used for the prediction, the predicted trajectory may simply be overlaid on the reference image. This overlay may be rendered in a different color or shade, for example, to be distinguished from untriggered predictions for the subsequent frames. Also, when uncertainty associated with the predicted trajectory becomes too high (e.g., exceeds a predetermined threshold), an alert may be provided to the user.

In block S419, it is determined whether navigation of the interventional device in the anatomical structure continues. This determination may be made, for example, when untriggered control inputs continue to be received, e.g., via the user interface, or when a predetermined target in the displayed image has not yet been reached. When it is determined that navigation is continuing (block S419: Yes), the process returns to block S413, where additional untriggered control inputs are received for controlling the robot for guiding future movement of the interventional device. Blocks S414 through S419 are then repeated to form a control loop for interactively guiding the interventional device using the robot. When it is determined that navigation is continuing (block S419: Yes), the process returns. When it is determined that navigation is not continuing (block S419: No), the process ends.

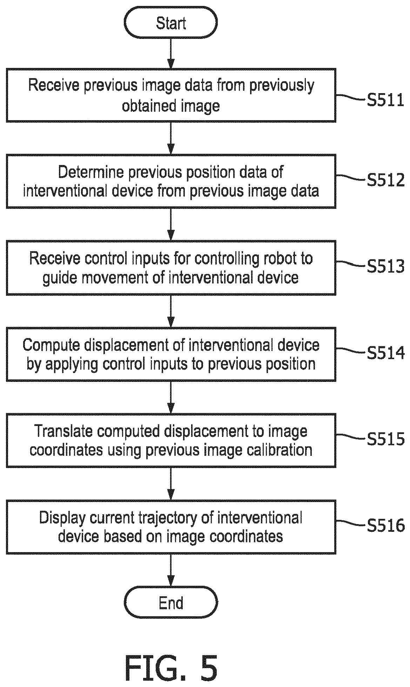

FIG. 5 is a flow diagram of a method of determining and visualizing trajectories of a robotically controlled interventional device on a display including a GUI, according to another representative embodiment. The method may be implemented by the system 100, discussed above, under control of the processor 120 executing instructions stored in the memory 130, for example. In the embodiment of FIG. 5, the trajectory of an interventional device may be estimated without use of a prediction model, thereby avoiding training and application of the neural network.

Referring to FIG. 5, the method includes receiving previous image data from a previously obtained image (e.g., from previous image module 133) of the anatomical structure in block S511. The previous image shows the interventional device and the anatomical structure in the subject at an earlier time. For example, the previous image may show the interventional device at time t=0, while the interventional device is currently at a time t=n, where n>0.

In block S512, previous position data of the interventional device is determined from the previous image data. The previous position data provides a previous position and a trajectory of the interventional device in the previous image. The previous position data may be determined by applying known segmentation techniques to the previous image, for example. The trajectory of the interventional device in particular may be determined by comparing the previous position of the interventional device in the previous image to earlier images of the interventional device. Here, the previous position describes how the interventional device is positioned in the context of the previous image, providing spatial context for the interventional device. The previous trajectory describes how the device moves across multiple previous images, providing temporal context for the interventional device.

In block S513, control inputs are received via the control input module (e.g., control input module 132) for controlling the robot to guide movement of the interventional device from the previous position. The control inputs include user inputs and state information of the robotic system. The control inputs may be triggered control inputs resulting in actual movement of the interventional device.

In block S514, displacement of the interventional device is computed by applying the received control inputs to the previous position data from the previous image. The displacement is from the position of the interventional device in the previous position provided by the previous position data to a displaced position in accordance with the control inputs.

In block S515, the computed displacement of the interventional device is translated to image coordinates using previous image calibration of the previous image. For example, in a case where the interventional device may be tracked using some technology that does not involve segmenting the interventional device in image space to provide position and/or trajectory information, tracking data may be in a different coordinate space compared to the image data, thus requiring calibration. However, the calibration information may be used in subsequent images.

In block S516, a current trajectory of the interventional device is displayed based on the image coordinates of the displacement. The current trajectory is displayed overlaid on the displayed previous image. In this manner, movement of the interventional device along the current trajectory is shown on the previous image, so that additional imaging is not necessary during the interventional procedure.

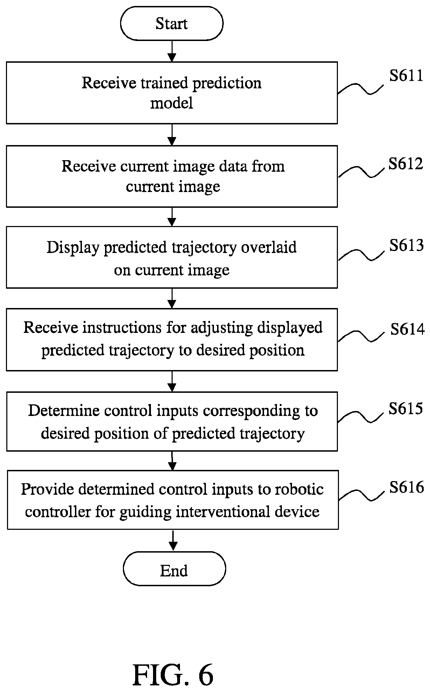

FIG. 6 is a flow diagram of a method of controlling a robotically controlled interventional device by adjusting visualized trajectories of the interventional device on a display including a GUI, according to another representative embodiment. The method may be implemented by the system 100, discussed above, under control of the processor 120 executing instructions stored in the memory 130, for example. The embodiment shown in FIG. 6 is essentially the reverse of the embodiment shown in FIG. 4, where the robot is controlled automatically by the robotic controller in response to instructions by the user that adjust displayed trajectories on the display.

Referring to FIG. 6, the method includes receiving a prediction model in block S611, such as a neural network model, for example. The prediction model has been trained with regard to determining control inputs for controlling the robot to guide the interventional device along a given predicted trajectory. The training may be based on training data from previous images of the interventional device and corresponding control inputs to the robot for guiding movement of the interventional device as shown in the previous images. The training may be further based on training data from current images of the interventional device and corresponding control inputs to the robot for guiding movement of the interventional device as shown in the current images.

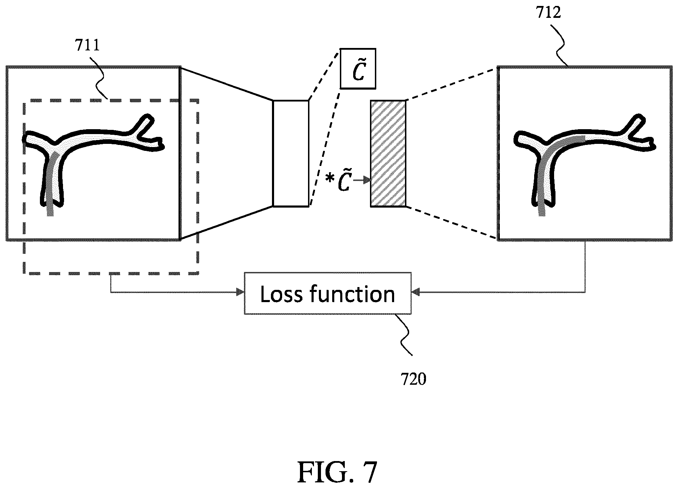

FIG. 7 is a simplified block diagram showing an example implementation of training an encoder-decoder neural network architecture, according to a representative embodiment. The encoder-decoder neural network architecture may be similar to the neural network described above with reference to FIG. 3, where the current image is input into an encoder portion of the neural network, which generates a reduced dimension latent representation of the input image. The latent representation is additionally used to estimate untriggered control inputs which can then be applied to the latent representation to produce a transformed latent representation. The transformed latent representation may be decoded to generate a predicted image containing the predicted trajectory of the interventional device as before.

Referring to FIG. 7, training image 711 may be used as a ground truth image, to which estimated control inputs have been applied. During training of the neural network, the predicted image 712 may be compared to training image 711, for instance, the predicted image 712 resulting from application of the estimated control inputs *C̃* on the current position of the interventional device. The comparison may be performed using a loss function 720, as discussed above, and the losses are used to perform gradient descent to update neural network parameters over multiple iterations in order to minimize the computed loss functions. When the corresponding control inputs are known, then the estimated control inputs *C̃* may also be compared with the known or ground truth corresponding control inputs. Both comparisons may be performed using the loss function 720, as discussed above, and a combination of the losses (e.g., average or weighted average) are used to perform gradient descent to update neural network parameters over multiple iterations in order to minimize the computed loss function.

During inference, when future image or control inputs are not known, the desired future trajectory of the interventional device may be specified by the user by drawing the desired trajectory on the GUI, for example. The estimated control inputs *C̃* may be fined-tuned during inference, as well by computing the transformed latent representation and decoding it to generate the predicted image containing the predicted trajectory of the interventional device and comparing it to the user specified desired future trajectory of the device. Comparisons may be performed using loss functions as described above, and the losses may be used to perform gradient descent as described above.

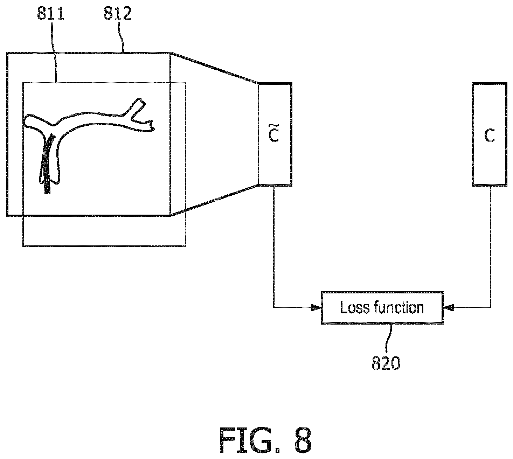

FIG. 8 is a simplified block diagram showing another example implementation of training an encoder-decoder neural network architecture, according to a representative embodiment. Referring to FIG. 8, the current image 812 and the image 811 containing the desired future trajectory of the interventional device (e.g., as indicated by the user drawing on the GUI) may both be input into a neural network (e.g., as separate channels) that directly estimates the untriggered control inputs that would transform the interventional device in the current image to the desired future trajectory. In this implementation, no fine-tuning is required during inference using a trained network. During training of the neural network, the estimated control inputs *C̃* are compared with known or ground truth corresponding control inputs C. Comparison and gradient descent are performed as specified above.

Referring again to FIG. 6, in block S612, current image data is received from a current image (e.g., from current image module 131) of the anatomical structure. The current image shows the anatomical structure in the subject, e.g., obtained by the imaging device 160, and the current position of the interventional device within the anatomical structure.

In block S613, a predicted trajectory of the interventional device is displayed on overlaid on the current image. The predicted trajectory of the interventional device is predicted by applying the current image data and the current control inputs to the trained prediction model, as discussed above. Along with the predicted trajectory, the trained prediction model may also provide estimated uncertainty associated with the predicted trajectory, which may also be displayed.

In block S614, instructions are received from the user to adjust the displayed predicted trajectory to a desired position. For example, the GUI may include a touchscreen that enables the user to drag the predicted trajectory to the desired position on the display screen. Or, as another example, the user may use a mouse to click and drag the predicted trajectory to the desired position.

In block S615, control inputs corresponding to the desired position of the predicted trajectory are determined by applying image coordinates of the predicted trajectory in the desired position to the prediction model, as described above.

In block S616, the determined control inputs are provided to the robotic controller, which automatically controls the robot to guide the interventional device along the desired predicted trajectory in response to the determined control inputs.

In various embodiments, the prediction model may be implemented using different architectures of encoder-decoder neural network models, for example, which may incorporate convolutional layers and LSTM blocks, for example. Generally, the current image data is input into the encoder portion of the encoder-decoder neural network model, which generates a reduced dimension latent representation of the input image. The untriggered control inputs may be input into the encoder portion or applied to the latent representation to produce a transformed latent representation. The encoded image data and the (encoded) control inputs may be decoded using the decoder portion of the encoder-decoder neural network model to generate the predicted image containing the predicted trajectory of the interventional device.

In this context, FIG. 9 is a simplified block diagram of an encoder-decoder neural network model configured to predict device trajectory with dimension preserving architecture, according to a representative embodiment, where image data and control inputs are input to the encoder portion. FIG. 10 is a simplified block diagram of an encoder-decoder neural network model configured to predict device trajectory with dimension varying architecture, according to a representative embodiment, where image data is input to the encoder portion and control inputs are input following the encoding of the image data.

Referring to FIG. 9, encoder-decoder neural network model 900 includes encoder 910, decoder 920, and latent state 905 between the encoder 910 and the decoder 920. The encoder 910 includes LSTM blocks 911, 912 and 913, and the decoder 920 includes LSTM blocks 921, 922 and 923, all of which are configured to capture temporal dependencies. The encoder-decoder neural network model 900 may therefore be referred to as an LSTM neural network model. The encoder-decoder neural network model 900 is referred to as dimension preserving because feature layers after each of the LSTM blocks 911-913 and the LSTM blocks 921-923 have the same size as the input data, indicated by the matching block shapes. Of course, the functionality of the LSTM blocks 911-913 and 921-923 may be provided by other types of neural network, such as an RNN, a convolutional neural network, CNN, or a transformer, without departing from the scope of the present teachings.

As shown, the encoder 910 receives as input both the image data (ID) (e.g., previous image data) showing the interventional device in the anatomical structure and the corresponding control inputs (CI) (e.g., previous and/or present control inputs) to be provided to the robot for guiding movement of the interventional device at the input level. That is, the image data and the corresponding control inputs are fused together or combined at the earliest layer of the neural network model. The latent state 905 indicates the hidden message following encoding of all the image data and the corresponding control inputs. The decoder 920 decodes data in the latent state to convert the encoded image data and control inputs to determine the predicted trajectory of the interventional device, which is output from the encoder-decoder neural network model 900. The image data for each of multiple images and control input corresponding to that image data are input to the LSTM blocks 911-913 in the encoder 910. With regard to the LSTM blocks 921-923 in the decoder 920, decoded data is consecutively input from a previous LSTM block, and consecutively output to the next LSTM block.

Referring to FIG. 10, encoder-decoder neural network model 1000 includes encoder 1010, decoder 1020, and latent state 1005 between the encoder 1010 and the decoder 1020. The encoder-decoder neural network model 1000 also includes fully connected layers 1030, LSTM encoder 1040 and combiner 1045. The encoder 1010 includes LSTM blocks 1011, 1012 and 1013, and the decoder 1020 includes LSTM blocks 1021, 1022 and 1023. The encoder-decoder neural network model 1000 may therefore be referred to as an LSTM neural network model. The encoder-decoder neural network model 1000 is referred to as dimension varying because the dimension of the input data is reduced gradually in the encoder 1010 until reaching a bottleneck layer, indicated by the narrowing dimensions of each of the LSTM blocks 1011-1013 from bottom to top. Conversely, the dimension of the latent state data is increased gradually from the bottleneck layer in the decoder 1020 back to the original input dimensions, indicated by the widening dimensions of each of the LSTM blocks 1021-1023 from bottom to top.

As shown, the encoder 1010 receives as input only the image data (ID) (e.g., previous image data) showing the anatomical structure at the input level, where the interventional device may also be visible in the image data. The latent state 1005 indicates the hidden message following encoding of all the image data by the encoder 1010. The control inputs (CI) (e.g., previous control inputs) corresponding to the image data are input to the fully connected layers 1030. The fully connected layer(s) is configured to apply linear and non-linear weights to the robot control inputs. In this case, the fully connected layer(s) allows the transformation of robot control data to a representation that can be merged with the image data at the latent layers, and compensate for the mismatch in dimension between the image and the robot control data. The output of the fully connected layers 1030 is input to the LSTM encoder 1040, which is configured to capture temporal dependencies, similar to the encoder 1010 discussed above, although without convolutional layers. The combiner 1045 combines the output of the LSTM encoder 1040 with the data in the latent state following the encoder 1010, and the combination is input to the decoder 1020. In alternative embodiments, the control inputs are combined with the data in the latent state at different stages of decoding by the decoder 1020, as indicated by the dashed arrow from the LSTM encoder 1040. This allows the encoder-decoder neural network model 1000 to learn dependencies between robotic control and image data at various scales and steps, resulting in more accurate predictions.

The decoder 1020 iteratively decodes data in the latent state to convert the combined encoded image data and control inputs to determine the predicted trajectory of the interventional device, which is output from the encoder-decoder neural network model 1000. The image data for each of multiple images and control input corresponding to that image data are input to the LSTM blocks 1011-1013 in the encoder 1010. With regard to the LSTM blocks 1021-1023 in the decoder 1020, decoded data is consecutively input from a previous LSTM block, and consecutively output to the next LSTM block.

In the embodiments discussed above, the predicted trajectory is determined for the future using current actual images (e.g., X-ray images, live fluoroscopy images) continuously acquired during the interventional procedure. However, in alternative embodiments, the images may be acquired discontinuously (e.g., periodically at a low frame rate or intermittently), in which case the navigation of the interventional device would need to be performed without actual images during the periods between image acquisitions. That is, when the images are acquired discontinuously, there are periods during which the robot is moving the interventional device within the anatomical structure without new incoming image data from the imaging device. To address this situation, the alternative embodiments provide prediction of the trajectory of the interventional device, although the prediction is of the current trajectory and/or position based on previous image data as opposed to prediction of the future trajectory and/or position based on current image data. In this case, the control input for controlling the robot to guide the interventional device is not untriggered, as in previous embodiments, but rather is triggered and applied in order to move the interventional device up until the present time.

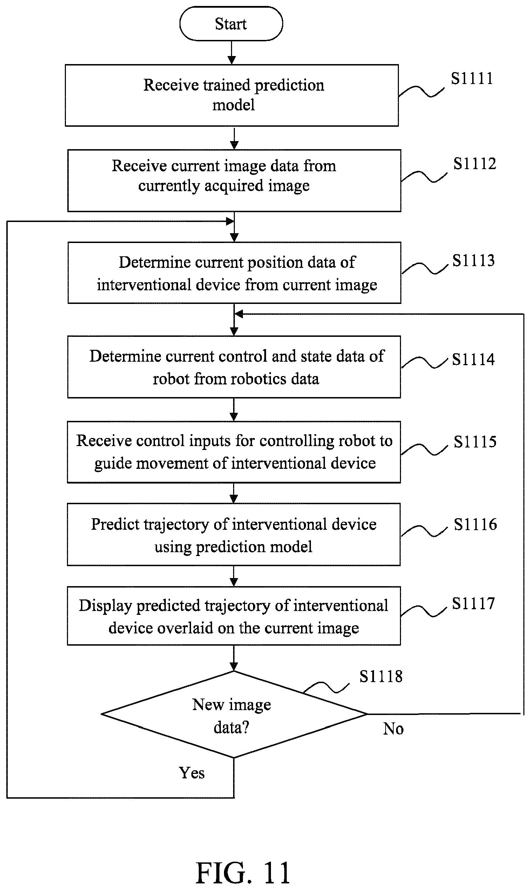

FIG. 11 is a flow diagram of a method of estimating and visualizing trajectories of a robotically controlled interventional device using discontinuous imaging, according to a representative embodiment. The method may be implemented by the system 100, discussed above, under control of the processor 120 executing instructions stored as the various software modules in the memory 130, for example. In the embodiment of FIG. 11, the trajectory of an interventional device may be estimated from a current image using a neural network model, thereby continued navigation of the interventional device via the robot without updating the images.

Referring to FIG. 11, the method includes receiving a prediction model in block S1111, where the prediction model has been previously trained with regard to predicting trajectories of the interventional device and associated uncertainties. The prediction model may be a neural network model, for example. The training may be based on training data from previous images of the interventional device and corresponding control inputs to the robot for guiding movement of the interventional device as shown in the previous images. The training may be further based on training data from current images of the interventional device and corresponding control inputs to the robot for guiding movement of the interventional device as shown in the current images.

In block S1112, current image data from a current image (e.g., from current image module 131) is received from a medical imaging device (e.g., imaging device 160), where the current image is displayed on a display (e.g., display 124). The current image shows the interventional device and the anatomical structure in the subject.

In block S1113, current position data of the interventional device is determined from the current image data. The current position data may be determined by applying known image analysis/processing techniques to the current image, for example. The current position data indicates how the interventional device is positioned in the context of the current image, providing spatial context for the interventional device.

In block S1114, current control and state data of the robot are determined using robotics data from the robotic system. The robotics data indicates positions and orientations of the robot while controlling the interventional device, and may include kinematics data, joint information, encoder information, velocities, accelerations, end-effector position, force, torque, and/or ranges and limits, for example. The current control and state data of the robot may be associated with the position data of the interventional device to determine the position of the robot when the interventional device is in its current position.

In block S1115, control inputs are received via the control input module (e.g., control input module 132) for controlling the robot to guide movement of the interventional device from the current position. The control inputs include user inputs and state information of the robotic system. The control inputs are triggered control inputs resulting in actual movement of the interventional device, and are intended to navigate the interventional device through the anatomical structure. The user inputs may result from movement by the user of one or more interface devices, such as a mouse, a keyboard, a trackball, a joystick, a microphone, a video camera, a touchpad, a touchscreen, for example.

In block S1116, a trajectory of the interventional device in the current image is predicted relative to the current position data of the interventional device by applying the current image data and the control inputs to the trained prediction model. The trained prediction model may also estimate uncertainty associated with the predicted trajectory, as discussed above. In an embodiment, the control inputs, the predicted trajectory and the associated uncertainty may be used to further train the prediction model on-the-fly.

In block S1117, the predicted trajectory of the interventional device is displayed on the display overlaid on the current image. Since the current image has not been updated, it is assumed that actual trajectory of the interventional device is following the predicted trajectory, as displayed, even though the actual position of the interventional device cannot be seen by the user. The user may adjust the user input in real time to alter the course of the predicted trajectory as needed to maintain safe navigation of the interventional device relative to the displayed current image. In an embodiment, the estimated uncertainty associated with the predicted trajectory may also be displayed over laid on the current image.

In block S1118, it is determined whether new current image data from a newly acquired current image has been received from the medical imaging device, where the new current image shows the interventional device in a new (updated) position within the anatomical structure in the subject. When new current image data has been received (block S1118: Yes), the new current image is displayed on the display, and the process returns to block S1113 for determining new current position data of the interventional device at its new position. Blocks S1114 to S1118 are then repeated based on the new current position data. When new current image data has not been received (block S1118: No), the same current image of the interventional device and the anatomical structure continues to be displayed, and the process returns to block S1114 for determining updated current control and state data of the robot. Blocks S1115 to S1118 are then repeated. The predicted trajectory of the interventional may change to reflect any changes to the user input.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although estimating and visualizing trajectories of a robotically controlled interventional device on a display has been described with reference to exemplary embodiments, it is understood that the words that have been used are words of description and illustration, rather than words of limitation. Although estimating and visualizing trajectories of a robotically controlled interventional device on a display has been described with reference to particular means, materials and embodiments, it is not intended to be limited to the particulars disclosed; rather the estimating and visualizing trajectories of a robotically controlled interventional device on a display extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive. Thus, to the maximum extent allowed by law, the scope of the present disclosure is to be determined by the broadest permissible interpretation of the following claims and their equivalents and shall not be restricted or limited by the foregoing detailed description.

## Claims

1. A system (100) for estimating and visualizing trajectories of an interventional device guided by a robot and configured for insertion into an anatomical structure of a subject, the system comprising:
at least one processor (120); and
a non-transitory memory (130) storing machine executable instructions that, when executed by the at least one processor, cause the at least one processor to:
receive image data (S412) from a current image of the anatomical structure, the current image showing a current position of the interventional device with respect to the anatomical structure;
receive at least one untriggered control input (S413) for controlling the robot to guide future movement of the interventional device from the current position;
predict at least one trajectory of the interventional device (S414) in the current image by providing the image data and the at least one untriggered control input to a model, wherein the model is configured to predict trajectories of the interventional device based on first input of image data of the interventional device in the anatomical structure and on second input of corresponding control inputs to the robot for guiding movement of the interventional device as shown in the image data of the first input; and
provide a trigger command for triggering the at least one untriggered control input to control the robot to guide movement of the interventional device according to the at least one triggered control input when the predicted trajectory is determined to be acceptable.

2. The system of claim 1, wherein the executed instructions further cause the at least one processor to:
automatically determine whether a predicted trajectory is acceptable.

3. The system of claim 2, wherein automatically determining whether the predicted trajectory is acceptable comprises:
capturing a shape of the predicted trajectory to provide a segmented predicted trajectory;
comparing the segmented predicted trajectory to a predetermined desired trajectory; and
determining that the predicted trajectory is acceptable when the segmented predicted trajectory substantially matches the predetermined desired trajectory.

4. The system of claim 2, wherein automatically determining whether the predicted trajectory is acceptable comprises:
capturing a shape of the predicted trajectory to provide a segmented predicted trajectory;
comparing the segmented predicted trajectory to a predetermined set of rules for navigating the interventional device through the anatomical structure; and
determining that the predicted trajectory is acceptable when the segmented predicted trajectory substantially matches the predetermined set of rules.

5. The system of claim 1 or 2, wherein the executed instructions further cause the at least one processor to:
provide a user interface (128) configured for allowing a user to send said trigger command.

6. The system of claim 5, wherein the user interface is further configured to provide to the user information regarding the at least one predicted trajectory to assist the user in deciding whether a predicted trajectory is acceptable, and thereby assisting the user to provide the trigger command.

7. The system of any of preceding claims, wherein the executed instructions further cause the at least one processor to:
receive at least one new untriggered control input for controlling the robot to alternatively guide the future movement of the interventional device from the current position when the trigger command is not provided.

8. The system of any of preceding claims, wherein said model is a trained model configured to predict trajectories of the interventional device based on training data from previous images of the interventional device in the anatomical structure and corresponding control inputs to the robot for guiding movement of the interventional device as shown in the previous images.

9. The system of any of preceding claims, wherein the executed instructions further cause the at least one processor to:
perform a training of said model with regard to predicting trajectories of the interventional device based on training data from at least one of previous images of the interventional device in the anatomical structure and the received image data and at least one of previous control inputs corresponding to the previous images and the at least one untriggered control input to the robot for guiding movement of the interventional device as shown in the previous images.

10. The system of any of preceding claims, wherein said model is a convolutional-long-short term memory (LSTM) neural network model (900, 1000).

11. The system of claim 10, wherein the LSTM neural network model is configured with dimension preserving architecture to predict the at least one trajectory of the interventional device, wherein the first input of the image data and the second input of the corresponding control inputs are combined at an earliest layer of an encoder (910) of the LSTM neural network model.

12. The system of claim 10, wherein the LSTM neural network model is configured with dimension varying architecture to predict the at least one trajectory of the interventional device, wherein the first input of the image data is input at an earliest layer of an encoder (1010) of the LSTM neural network model, and the second input of the corresponding control inputs is input at or after a latent state (1005) between the encoder and a decoder (1020) of the LSTM neural network model.

13. The system of any of preceding claims, wherein the model is further configured to predict trajectories of the interventional device further based on an input of shape data of the interventional device shown in the image data of the first input.

14. The system of any of preceding claims, wherein the model is further configured to process temporal sequences of imaging data such that the trajectories are progressively predicted over time, when the interventional device moves.

15. A system (100) for estimating and visualizing trajectories of an interventional device guided by a robot and configured for insertion into an anatomical structure of a subject, the system comprising:
at least one processor (120); and
a non-transitory memory (130) storing machine executable instructions that, when executed by the at least one processor, cause the at least one processor to:
receive a model (S411) configured to predict trajectories of the interventional device based on data from images of the interventional device in the anatomical structure and corresponding control inputs to the robot for guiding movement of the interventional device as shown in the images;
receive image data (S412) from a current image of the anatomical structure, the current image showing a current position of the interventional device with respect to the anatomical structure;
receive untriggered control inputs (S413) for controlling the robot to guide future movement of the interventional device from the current position;
predict a trajectory of the interventional device (S414) in the current image by applying the image data and the untriggered control inputs to the model;
display the predicted trajectory of the interventional device (S415) overlaid on the current image of the anatomical structure for a user to determine whether the predicted trajectory is acceptable (S416);
receive a trigger command (S417) for triggering the untriggered control inputs to control the robot to guide movement of the interventional device according to the triggered control inputs when the predicted trajectory is determined to be acceptable; and
receive new untriggered control inputs (S413) for controlling the robot to alternatively guide the future movement of the interventional device from the current position when the predicted trajectory is determined to be not acceptable.

16. A computer program product comprising instructions which, when executed on a processor, cause the processor to carry out the following method steps of a method of estimating and visualizing trajectories of an interventional device guided by a robot and configured for insertion into an anatomical structure of a subject:
performing training of a neural network model with regard to predicting trajectories of the interventional device based on training data from previous images of the interventional device in the anatomical structure and corresponding control inputs to the robot for guiding movement of the interventional device as shown in the previous images;
receiving image data (S412) from at least one image of the anatomical structure, the at least one image showing a current position of the interventional device with respect to the anatomical structure;
receiving untriggered control inputs (S413) for controlling the robot to guide future movement of the interventional device from the current position;
predicting a trajectory of the interventional device (S414) in the at least one image by applying the image data and the untriggered control inputs to the trained neural network model;
displaying the predicted trajectory of the interventional device (S415) overlaid on the at least one image of the anatomical structure;
triggering the untriggered control inputs (S417) to control the robot to guide movement of the interventional device according to the triggered control inputs when the predicted trajectory is determined to be acceptable (S416); and
receiving new untriggered control inputs (S413) for controlling the robot to alternatively guide the future movement of the interventional device from the current position when the predicted trajectory is determined to be not acceptable.

## Patentansprüche

1. System (100) zum Schätzen und Visualisieren von Bewegungsbahnen einer Eingriffsvorrichtung, welche von einem Roboter geführt wird und zum Einsetzen in eine anatomische Struktur eines Subjekts konfiguriert ist, wobei das System umfasst:
zumindest einen Prozessor (120); und
einen nichtflüchtigen Speicher (130), welcher maschinenausführbare Anweisungen speichert, welche, wenn sie von dem zumindest einen Prozessor ausgeführt werden, den zumindest einen Prozessor dazu veranlassen:
Bilddaten von einem aktuellen Bild der anatomischen Struktur zu empfangen (S412), wobei das aktuelle Bild eine aktuelle Position der Eingriffsvorrichtung in Bezug auf die anatomische Struktur zeigt;
zumindest eine nicht ausgelöste Steuereingabe zum Steuern des Roboters zu empfangen (S413), um zukünftige Bewegung der Eingriffsvorrichtung von der aktuellen Position aus zu führen.
zumindest eine Bewegungsbahn der Eingriffsvorrichtung in dem aktuellen Bild durch Bereitstellen der Bilddaten und der zumindest einen nicht ausgelösten Steuereingabe an ein Modell vorherzusagen (S414), wobei das Modell dazu konfiguriert ist, Bewegungsbahnen der Eingriffsvorrichtung basierend auf einer ersten Eingabe von Bilddaten der Eingriffsvorrichtung in der anatomischen Struktur und einer zweiten Eingabe von entsprechenden Steuereingaben an den Roboter zum Führen der Bewegung der Eingriffsvorrichtung, wie in den Bilddaten der ersten Eingabe gezeigt, vorherzusagen; und
Bereitstellen eines Auslösebefehls zum Auslösen der zumindest einen nicht ausgelösten Steuereingabe, um den Roboter so zu steuern, dass er die Bewegung der Eingriffsvorrichtung entsprechend der zumindest einen ausgelösten Steuereingabe führt, wenn die vorhergesagte Bewegungsbahn als akzeptabel bestimmt wird.

2. System nach Anspruch 1, wobei die ausgeführten Anweisungen den zumindest einen Prozessor weiter dazu veranlassen:
automatisch zu bestimmen, ob eine vorhergesagte Bewegungsbahn akzeptabel ist.

3. System nach Anspruch 2, wobei automatisches Bestimmen, ob die vorhergesagte Bewegungsbahn akzeptabel ist, umfasst:
Erfassen einer Form der vorhergesagten Bewegungsbahn, um eine segmentierte vorhergesagte Bewegungsbahn bereitzustellen;
Vergleichen der segmentierten vorhergesagten Bewegungsbahn mit einer vorbestimmten gewünschten Bewegungsbahn; und
Bestimmen, dass die vorhergesagte Bewegungsbahn akzeptabel ist, wenn die segmentierte vorhergesagte Bewegungsbahn im Wesentlichen mit der vorbestimmten gewünschten Bewegungsbahn übereinstimmt.

4. System nach Anspruch 2, wobei automatisches Bestimmen, ob die vorhergesagte Bewegungsbahn akzeptabel ist, umfasst:
Erfassen einer Form der vorhergesagten Bewegungsbahn, um eine segmentierte vorhergesagte Bewegungsbahn bereitzustellen;
Vergleichen der segmentierten vorhergesagten Bewegungsbahn mit einem vorbestimmten Satz von Regeln zum Navigieren der Eingriffsvorrichtung durch die anatomische Struktur; und
Bestimmen, dass die vorhergesagte Bewegungsbahn akzeptabel ist, wenn die segmentierte vorhergesagte Bewegungsbahn im Wesentlichen mit dem vorbestimmten Satz von Regeln übereinstimmt.

5. System nach Anspruch 1 oder 2, wobei die ausgeführten Anweisungen den zumindest einen Prozessor weiter dazu veranlassen:
eine Benutzerschnittstelle (128) bereitzustellen, die dazu konfiguriert ist, einem Benutzer zu ermöglichen, den Auslösebefehl zu senden.

6. System nach Anspruch 5, wobei die Benutzerschnittstelle weiter dazu konfiguriert ist, dem Benutzer Informationen hinsichtlich der zumindest einen vorhergesagten Bewegungsbahn bereitzustellen, um den Benutzer bei der Entscheidung zu unterstützen, ob eine vorhergesagte Bewegungsbahn akzeptabel ist, und um den Benutzer dadurch bei der Bereitstellung des Auslösebefehls zu unterstützen.

7. System nach einem der vorstehenden Ansprüche, wobei die ausgeführten Anweisungen den zumindest einen Prozessor weiter dazu veranlassen:
zumindest eine neue nicht ausgelöste Steuereingabe zum Steuern des Roboters zu empfangen, um die zukünftige Bewegung der Eingriffsvorrichtung von der aktuellen Position aus alternativ zu führen, wenn der Auslösebefehl nicht bereitgestellt wird.

8. System nach einem der vorstehenden Ansprüche, wobei das Modell ein trainiertes Modell ist, welches dazu konfiguriert ist, Bewegungsbahnen der Eingriffsvorrichtung basierend auf Trainingsdaten aus vorherigen Bildern der Eingriffsvorrichtung in der anatomischen Struktur und von entsprechenden Steuereingaben an den Roboter zum Führen der Bewegung der Eingriffsvorrichtung, wie in den vorherigen Bildern gezeigt, vorherzusagen.

9. System nach einem der vorstehenden Ansprüche, wobei die ausgeführten Anweisungen den zumindest einen Prozessor weiter dazu veranlassen:
ein Training des Modells im Hinblick auf Vorhersagen von Bewegungsbahnen der Eingriffsvorrichtung basierend auf Trainingsdaten aus zumindest einem der vorherigen Bilder der Eingriffsvorrichtung in der anatomischen Struktur und den empfangenen Bilddaten und zumindest einer der vorherigen Steuereingaben, welche den vorherigen Bildern entsprechen, und der zumindest einen nicht ausgelösten Steuereingabe an den Roboter zum Führen der Bewegung der Eingriffsvorrichtung, wie in den vorherigen Bildern gezeigt, durchzuführen.

10. System nach einem der vorstehenden Ansprüche, wobei das Modell ein Convolutional-Long-Short-Term Memory (LSTM)-Neuralnetzwerkmodell (900, 1000) ist.

11. System nach Anspruch 10, wobei das LSTM-Neuralnetzwerkmodell mit einer dimensionserhaltenden Architektur konfiguriert ist, um die zumindest eine Bewegungsbahn der Eingriffsvorrichtung vorherzusagen, wobei die erste Eingabe der Bilddaten und die zweite Eingabe der entsprechenden Steuereingaben in einer frühesten Schicht eines Encoders (910) des LSTM-Neuralnetzwerkmodells kombiniert werden.

12. System nach Anspruch 10, wobei das LSTM-Neuralnetzwerkmodell mit einer dimensionsvariablen Architektur konfiguriert ist, um die zumindest eine Bewegungsbahn der Eingriffsvorrichtung vorherzusagen, wobei die erste Eingabe der Bilddaten in einer frühesten Schicht eines Encoders (1010) des LSTM-Neuralnetzwerkmodells eingegeben wird und die zweite Eingabe der entsprechenden Steuereingaben in oder nach einem latenten Zustand (1005) zwischen dem Encoder und einem Decoder (1020) des LSTM-Neuralnetzwerkmodells eingegeben wird.

13. System nach einem der vorstehenden Ansprüche, wobei das Modell weiter dazu konfiguriert ist, Bewegungsbahnen der Eingriffsvorrichtung weiter auf Grundlage einer Eingabe von Formdaten der Eingriffsvorrichtung vorherzusagen, welche in den Bilddaten der ersten Eingabe gezeigt sind.

14. System nach einem der vorstehenden Ansprüche, wobei das Modell weiter dazu konfiguriert ist, zeitliche Sequenzen von Bildgebungsdaten so zu verarbeiten, dass die Bewegungsbahnen im Laufe der Zeit fortschreitend vorhergesagt werden, wenn sich die Eingriffsvorrichtung bewegt.

15. System (100) zum Schätzen und Visualisieren von Bewegungsbahnen einer Eingriffsvorrichtung, welche von einem Roboter geführt wird und zum Einsetzen in eine anatomische Struktur eines Subjekts konfiguriert ist, wobei das System umfasst:
zumindest einen Prozessor (120); und
einen nichtflüchtigen Speicher (130), welcher maschinenausführbare Anweisungen speichert, welche, wenn sie von dem zumindest einen Prozessor ausgeführt werden, den zumindest einen Prozessor dazu veranlassen:
ein Modell zu empfangen (S411), welches dazu konfiguriert ist, Bewegungsbahnen der Eingriffsvorrichtung basierend auf Daten aus Bildern der Eingriffsvorrichtung in der anatomischen Struktur und von entsprechenden Steuereingaben an den Roboter zum Führen der Bewegung der Eingriffsvorrichtung, wie in den Bildern gezeigt, vorherzusagen; und
Bilddaten von einem aktuellen Bild der anatomischen Struktur zu empfangen (S412), wobei das aktuelle Bild eine aktuelle Position der Eingriffsvorrichtung in Bezug auf die anatomische Struktur zeigt;
nicht ausgelöste Steuereingaben zum Steuern des Roboters zu empfangen (S413), um zukünftige Bewegung der Eingriffsvorrichtung von der aktuellen Position aus zu führen;
eine Bewegungsbahn der Eingriffsvorrichtung in dem aktuellen Bild durch Anwenden der Bilddaten und der nicht ausgelösten Steuereingaben an das Modell vorherzusagen (S414),
die vorhergesagte Bewegungsbahn der Eingriffsvorrichtung anzuzeigen (S415), welche das aktuelle Bild der anatomischen Struktur überlagert, damit ein Benutzer bestimmen kann, ob die vorhergesagte Bewegungsbahn akzeptabel ist (S416);
einen Auslösebefehl zum Auslösen der nicht ausgelösten Steuereingaben zu empfangen (S417), um den Roboter so zu steuern, dass er die Bewegung der Eingriffsvorrichtung entsprechend der ausgelösten Steuereingaben führt, wenn die vorhergesagte Bewegungsbahn als akzeptabel bestimmt wird; und
neue nicht ausgelöste Steuereingaben zum Steuern des Roboters zu empfangen (S413), um die zukünftige Bewegung der Eingriffsvorrichtung von der aktuellen Position aus alternativ zu führen, wenn die vorhergesagte Bewegungsbahn als nicht akzeptabel bestimmt wird.

16. Computerprogrammprodukt, welches Anweisungen umfasst, welche, wenn sie auf einem Prozessor ausgeführt werden, den Prozessor veranlassen, die folgenden Verfahrensschritte eines Verfahrens zum Schätzen und Visualisieren von Bewegungsbahnen einer Eingriffsvorrichtung auszuführen, welche von einem Roboter geführt wird und zum Einsetzen in eine anatomische Struktur eines Subjekts konfiguriert ist:
Durchführen von Training eines Neuralnetzwerkmodells in Bezug auf Vorhersagen von Bewegungsbahnen der Eingriffsvorrichtung basierend auf Trainingsdaten aus vorherigen Bildern der Eingriffsvorrichtung in der anatomischen Struktur und von entsprechenden Steuereingaben an den Roboter zum Führen der Bewegung der Eingriffsvorrichtung, wie in den vorherigen Bildern gezeigt;
Empfangen von Bilddaten (S412) von zumindest einem Bild der anatomischen Struktur, wobei das zumindest eine Bild eine aktuelle Position der Eingriffsvorrichtung in Bezug auf die anatomische Struktur zeigt;
Empfangen nicht ausgelöster Steuereingaben (S413) zum Steuern des Roboters, um zukünftige Bewegung der Eingriffsvorrichtung von der aktuellen Position aus zu führen;
Vorhersagen einer Bewegungsbahn der Eingriffsvorrichtung (S414) in dem zumindest einen Bild durch Anwenden der Bilddaten und der nicht ausgelösten Steuereingaben an das trainierte Neuralnetzwerkmodell;
Anzeigen der vorhergesagten Bewegungsbahn der Eingriffsvorrichtung (S415), welche das zumindest eine Bild der anatomischen Struktur überlagert;
Auslösen der nicht ausgelösten Steuereingaben (S417), um den Roboter so zu steuern, dass er die Bewegung der Eingriffsvorrichtung entsprechend der ausgelösten Steuereingaben führt, wenn die vorhergesagte Bewegungsbahn als akzeptabel bestimmt wird (S416); und
Empfangen neuer nicht ausgelöster Steuereingaben (S413) zum Steuern des Roboters, um die zukünftige Bewegung der Eingriffsvorrichtung von der aktuellen Position aus alternativ zu führen, wenn die vorhergesagte Bewegungsbahn als nicht akzeptabel bestimmt wird.

## Revendications

1. Système (100) pour estimer et visualiser des trajectoires d'un dispositif d'intervention guidé par un robot et configuré pour être inséré dans une structure anatomique d'un sujet, le système comprenant :
au moins un processeur (120) ; et
une mémoire non transitoire (130) stockant des instructions exécutables par machine qui, lorsqu'elles sont exécutées par le au moins un processeur, amènent le au moins un processeur à :
recevoir des données d'image (S412) d'une image actuelle de la structure anatomique, l'image actuelle représentant une position actuelle du dispositif d'intervention par rapport à la structure anatomique ;
recevoir la au moins une entrée de commande non déclenchée (S413) pour commander le robot pour guider un futur mouvement du dispositif d'intervention à partir de la position actuelle ;
prédire au moins une trajectoire du dispositif d'intervention (S414) dans l'image actuelle en fournissant les données d'image et la au moins une entrée de commande non déclenchée à un modèle, dans lequel le modèle est configuré pour prédire des trajectoires du dispositif d'intervention sur la base d'une première entrée de données d'image du dispositif d'intervention dans la structure anatomique et d'une seconde entrée d'entrées de commande correspondantes au robot pour guider un mouvement du dispositif d'intervention comme indiqué dans les données d'image de la première entrée ; et
fournir une commande de déclenchement pour déclencher la au moins une entrée de commande non déclenchée pour commander le robot pour guider un mouvement du dispositif d'intervention selon la au moins une entrée de commande déclenchée lorsqu'il est déterminé que la trajectoire prédite est acceptable.

2. Système selon la revendication 1, dans lequel les instructions exécutées amènent en outre le au moins un processeur à :
déterminer automatiquement si une trajectoire prédite est, ou non, acceptable.

3. Système selon la revendication 2, dans lequel la détermination automatique pour savoir si la trajectoire prédite est, ou non, acceptable, comprend :
la capture d'une forme de la trajectoire prédite pour fournir une trajectoire prédite segmentée ;
la comparaison de la trajectoire prédite segmentée à une trajectoire souhaitée prédéterminée ; et
la détermination que la trajectoire prédite est acceptable lorsque la trajectoire prédite segmentée correspond sensiblement à la trajectoire souhaitée prédéterminée.

4. Système selon la revendication 2, dans lequel la détermination automatique pour savoir si la trajectoire prédite est, ou non, acceptable, comprend :
la capture d'une forme de la trajectoire prédite pour fournir une trajectoire prédite segmentée ;
la comparaison de la trajectoire segmentée prédite à un ensemble prédéterminé de règles pour faire naviguer le dispositif d'intervention à travers la structure anatomique ; et
la détermination que la trajectoire prédite est acceptable lorsque la trajectoire prédite segmentée correspond sensiblement à l'ensemble prédéterminé de règles.

5. Système selon la revendication 1 ou 2, dans lequel les instructions exécutées amènent en outre le au moins un processeur à :
fournir une interface utilisateur (128) configurée pour permettre à un utilisateur d'envoyer ladite commande de déclenchement.

6. Système selon la revendication 5, dans lequel l'interface utilisateur est en outre configurée pour fournir, à l'utilisateur, des informations concernant la au moins une trajectoire prédite pour aider l'utilisateur à décider si une trajectoire prédite est, ou non, acceptable, et, ainsi, aider l'utilisateur à fournir la commande de déclenchement.

7. Système selon l'une quelconque des revendications précédentes, dans lequel les instructions exécutées amènent en outre le au moins un processeur à :
recevoir au moins une nouvelle entrée de commande non déclenchée pour commander le robot pour guider alternativement le futur mouvement du dispositif d'intervention à partir de la position actuelle lorsque la commande de déclenchement n'est pas fournie.

8. Système selon l'une quelconque des revendications précédentes, dans lequel ledit modèle est un modèle entraîné configuré pour prédire des trajectoires du dispositif d'intervention sur la base de données d'entraînement provenant de précédentes images du dispositif d'intervention dans la structure anatomique et d'entrées de commande correspondantes au robot pour guider un mouvement du dispositif d'intervention comme montré dans les précédentes images.

9. Système selon l'une quelconque des revendications précédentes, dans lequel les instructions exécutées amènent en outre le au moins un processeur à :
réaliser un entraînement dudit modèle en ce qui concerne la prédiction de trajectoires du dispositif d'intervention sur la base de données d'entraînement provenant d'au moins une de précédentes images du dispositif d'intervention dans la structure anatomique et des données d'image reçues et d'au moins une de précédentes entrées de commande correspondant aux précédentes images et de la au moins une entrée de commande non déclenchée au robot pour guider un mouvement du dispositif d'intervention comme montré dans les précédentes images.

10. Système selon l'une quelconque des revendications précédentes, dans lequel ledit modèle est un modèle de réseau neuronal à mémoire à long terme et à court terme convolutionnel (LSTM) (900, 1000).

11. Système selon la revendication 10, dans lequel le modèle de réseau neuronal à LSTM est configuré avec une architecture préservant les dimensions pour prédire la au moins une trajectoire du dispositif d'intervention, dans lequel la première entrée des données d'image et la seconde entrée des entrées de commande correspondantes sont combinées au niveau de la couche la plus ancienne d'un codeur (910) du modèle de réseau neuronal à LSTM.

12. Système selon la revendication 10, dans lequel le modèle de réseau neuronal à LSTM est configuré avec une architecture faisant varier les dimensions pour prédire la au moins une trajectoire du dispositif d'intervention, dans lequel la première entrée des données d'image est entrée au niveau de la couche la plus ancienne d'un codeur (1010) du modèle de réseau neuronal à LSTM, et la seconde entrée des entrées de commande correspondantes est entrée au moment d'un, ou après un, état latent (1005) entre le codeur et un décodeur (1020) du modèle de réseau neuronal à LSTM.

13. Système selon l'une quelconque des revendications précédentes, dans lequel le modèle est en outre configuré pour prédire des trajectoires du dispositif d'intervention en outre sur la base d'une entrée de données de forme du dispositif d'intervention représentées dans les données d'image de la première entrée.

14. Système selon l'une quelconque des revendications précédentes, dans lequel le modèle est en outre configuré pour traiter des séquences temporelles de données d'imagerie de telle sorte que les trajectoires soient progressivement prédites au fil du temps, lorsque le dispositif d'intervention se déplace.

15. Système (100) pour estimer et visualiser des trajectoires d'un dispositif d'intervention guidé par un robot et configuré pour être inséré dans une structure anatomique d'un sujet, le système comprenant :
au moins un processeur (120) ; et
une mémoire non transitoire (130) pour stocker des instructions exécutables par machine qui, lorsqu'elles sont exécutées par le au moins un processeur, amènent le au moins un processeur à :
recevoir un modèle (S411) configuré pour prédire des trajectoires du dispositif d'intervention sur la base de données provenant d'images du dispositif d'intervention dans la structure anatomique et d'entrées de commande correspondantes au robot pour guider un mouvement du dispositif d'intervention comme indiqué dans les images ;
recevoir des données d'image (S412) d'une image actuelle de la structure anatomique, l'image actuelle représentant une position actuelle du dispositif d'intervention par rapport à la structure anatomique ;
recevoir des entrées de commande non déclenchées (S413) pour commander le robot pour guider un futur mouvement du dispositif d'intervention à partir de la position actuelle ;
prédire une trajectoire du dispositif d'intervention (S414) dans l'image actuelle en appliquant les données d'image et les entrées de commande non déclenchées au modèle ;
afficher la trajectoire prédite du dispositif d'intervention (S415) superposée sur l'image actuelle de la structure anatomique pour qu'un utilisateur puisse déterminer si la trajectoire prédite est, ou non, acceptable (S416) ;
recevoir une commande de déclenchement (S417) pour déclencher les entrées de commande non déclenchées pour commander le robot pour guider un mouvement du dispositif d'intervention selon les entrées de commande déclenchées lorsqu'il est déterminé que la trajectoire prédite est acceptable ; et
recevoir de nouvelles entrées de commande non déclenchées (S413) pour commander le robot pour guider alternativement le futur mouvement du dispositif d'intervention à partir de la position actuelle lorsqu'il déterminé que la trajectoire prédite n'est pas acceptable.

16. Produit de programme informatique comprenant des instructions qui, lorsqu'elles sont exécutées sur un processeur, amènent le processeur à exécuter les étapes suivantes d'un procédé d'estimation et de visualisation de trajectoires d'un dispositif d'intervention guidé par un robot et configuré pour être inséré dans une structure anatomique d'un sujet :
la réalisation de l'entraînement d'un modèle de réseau neuronal en ce qui concerne la prédiction de trajectoires du dispositif d'intervention sur la base de données d'entraînement provenant de précédentes images du dispositif d'intervention dans la structure anatomique et d'entrées de commande correspondantes au robot pour guider un mouvement du dispositif d'intervention comme indiqué dans les images précédentes ;
la réception de données d'image (S412) d'au moins une image de la structure anatomique, la au moins une image représentant une position actuelle du dispositif d'intervention par rapport à la structure anatomique ;
la réception d'entrées de commande non déclenchées (S413) pour commander le robot pour guider un futur mouvement du dispositif d'intervention à partir de la position actuelle ;
la prédiction d'une trajectoire du dispositif d'intervention (S414) dans la au moins une image en appliquant les données d'image et les entrées de commande non déclenchées au modèle de réseau neuronal entraîné ;
l'affichage de la trajectoire prédite du dispositif d'intervention (S415) superposée sur la au moins une image de la structure anatomique ;
le déclenchement des entrées de commande non déclenchées (S417) pour commander le robot pour guider un mouvement du dispositif d'intervention selon les entrées de commande déclenchées lorsqu'il déterminé que la trajectoire prédite est acceptable (S416) ; et
la réception de nouvelles entrées de commande non déclenchées (S413) pour commander le robot pour guider alternativement le futur mouvement du dispositif d'intervention à partir de la position actuelle lorsqu'il est déterminé que la trajectoire prédite n'est pas acceptable.
